(19) Europäisches Patentamt
European Patent Office
Office européen des brevets

(11) Veröffentlichungsnummer: **0 006 180**
**B1**

(12) # EUROPÄISCHE PATENTSCHRIFT

(21) Anmeldenummer: **79101773.4**

(22) Anmeldetag: **05.06.79**

(51) Int. Cl.³: **C 07 C 119/10,**
C 07 C 45/00,
C 07 C 47/52//C07C93/14

(54) **3-Phenoxybenzylidenamine und 3-Benzylbenzylidenamine, ein Verfahren zu deren Herstellung sowie deren Verwendung zur Herstellung der entsprechenden Aldehyde.**

(30) Priorität: **08.06.78 CH 6289/78**
**10.04.79 CH 3406/79**

(43) Veröffentlichungstag der Anmeldung:
**09.01.80 Patentblatt 80/1**

(45) Bekanntmachung des Hinweises auf die Patenterteilung:
**11.02.81 Patentblatt 81/6**

(84) Benannte Vertragsstaaten:
**AT BE CH DE FR GB IT NL**

(56) Entgegenhaltungen:
**MONATSHEFTE FÜR CHEMIE, Band 67, 1936 Leipzig und Wien**
**G. LOCK et al. "Über Derivate des Phenyläthers" Seiten 24 bis 35**

(73) Patentinhaber: **CIBA-GEIGY AG**
**Patentabteilung Postfach**
**CH-4002 Basel (CH)**

(72) Erfinder: **Reinehr, Dieter, Dr.**
**Wolfsheule 10**
**D-7842 Kandern (DE)**

(72) Erfinder: **Gsell, Laurenz, Dr.**
**Maiengasse 56**
**CH-4056 Basel (CH)**

(74) Vertreter: **Zumstein sen., Fritz, Dr. et al,**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

3-Phenoxybenzylidenamine und 3-Benzylbenzylidenamine ein Verfahren zu deren Herstellung sowie deren Verwendung zur Herstellung der entsprechenden Aldehyde.

Die vorliegende Erfindung betrifft 3-Phenoxybenzylidenamine und 3-Benzylbenzylidenamine, ein Verfahren zu deren Herstellung sowie deren Verwendung zur Herstellung der entsprechenden Aldehyde.

Die erfindungsgemässen Verbindungen stellen wertvolle Ausgangsprodukte zur Herstellung von 3-Phenoxybenzaldehyden resp. 3-Benzylbenzaldehyden dar. Diese Aldehyden sind ihrerseits u.a. wertvolle Zwischenprodukte zur Herstellung von Pyrethroidartigen Schädlingsbekämpfungsmitteln, wie sie z.B. in der deutschen Patentschrift 2.231.312 beschrieben sind. Gemäss dieser Patentschrift kann der 3-Phenoxybenzaldehyd in Gegenwart von Natrium- oder Kaliumcyanid direkt mit Cyclopropancarbonsäurehalogeniden zu insektiziden Wirkstoffen umgesetzt werden, oder 3-Phenoxy-$\alpha$-cyanobenzylalkohol wird mit einer Cyclopropancarbonsäure oder einem reaktionsfähigen Derivat davon umgesetzt. Der 3-Phenoxy-$\alpha$-cyanobenzylalkohol seinerseits lässt sich durch Umsetzung von 3-Phenoxybenzaldehyd mit Natriumcyanid in Gegenwart von Essigsäure herstellen (vgl. auch schweizerische Patentschrift 589.051).

Nach den bisher bekanntgewordenen Verfahren, z.B. nach der Sommelet-Reaktion oder den in Monatscheft für Chemie, *67*, 24—35 (1936) und der deutschen Offenlegungsschrift 2.624.360 beschriebenen Verfahren, lässt sich beispielsweise der 3-Phenoxybenzaldehyd nicht ohne weiteres in der für die weitere Verwendung, z.B. zur Herstellung von insektiziden Wirkstoffen der vorerwähnten Art, erforderlichen Reinheit herstellen, so dass aufwendige Reinigungsoperationen notwendig sind [vgl. z.B. belgische Patentschrift 857.954]. Zudem müssen diese Verfahren teilweise unter rigorosen Reaktionsbedingungen durchgeführt werden oder sie sind hinsichtlich der Ausbeute unbefriedigend.

Durch die vorliegende Erfindung werden auf einfache Weise neue Zwischenprodukte zugänglich gemacht, die sich unter milden, umweltfreundlichen Reaktionsbedingungen mit hohen Ausbeuten in hochreine 3-Phenoxybenzaldehyde resp. 3-Benzylbenzaldehyde überführen lassen, der direkt für weitere Umsetzungen verwendet werden können.

Die erfindungsgemässen Verbindungen entsprechen der Formel

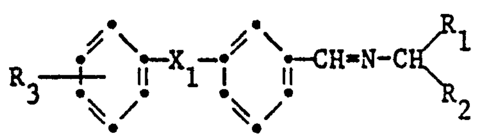

(I)

worin $R_1$ Wasserstoff oder Alkyl und
$R_2$ Alkyl bedeuten, wobei durch
$R_1$ und $R_2$ dargestellte Alkylgruppen zusammen nicht mehr als 12 C-Atome aufweisen oder
$R_1$ und $R_2$ zusammen Alkylen mit 4—11 C-Atomen darstellen,
$R_3$ Wasserstoff, Halogen, Methyl oder Methoxy und
$X_1$ Sauerstoff oder $-CH_2-$ bedeuten.

Unter Halogen bei $R_3$ ist Fluor, Chlor, Brom oder Jod, insbesondere aber Fluor oder Chlor, zu verstehen.

Durch $R_1$ und $R_2$ dargestellte Alkylgruppen können geradkettig oder verzweigt sein und weisen zusammen bevorzugt nicht mehr als 8 und insbesondere nicht mehr als 6 C-Atome auf. Beispiele definitionsgemässer Alkylgruppen $R_1$ und $R_2$ seien genannt: die Methyl-, Aethyl-, n-Propyl-, Isopropyl-, n-Butyl-, sek-Butyl-, tert-Butyl-, n-Pentyl-, 3-Pentyl-, n-Hexyl-, 2-Heptyl-, 3-Heptyl-, n-Octyl-, n-Decyl- und n-Dodecylgruppe.

Bilden $R_1$ und $R_2$ zusammen eine Alkylenkette, so weist diese bevorzugt 4—7 und insbesondere 4 oder 5 C-Atome auf.

Bevorzugt sind Verbindungen der Formel I, worin $R_1$ Wasserstoff oder Alkyl und $R_2$ Alkyl bedeuten und $R_1$ und $R_2$ zusammen nicht mehr als 6 C-Atome aufweisen, oder worin $R_1$ und $R_2$ zusammen Tetramethylen oder Pentamethylen und $R_3$ Wasserstoff, Fluor oder Chlor darstellen. Ganz besonders bevorzugt sind Verbindungen der Formel I, worin $R_1$ Wasserstoff und $R_2$ $\alpha$-verzweigtes Alkyl mit bis zu 6 C-Atomen, vor allem Isopropyl und $R_3$ Wasserstoff oder p-Fluor bedeuten.

Die Verbindungen der Formel I können nach dem erfindungsgemässen Verfahren auf einfache, wirtschaftliche Weise dadurch hergestellt werden, dass man 3-Phenoxybenzonitrile oder 3-Benzylbenzonitrile katalytisch zu 3-Phenoxybenzylaminen, resp. 3-Benzylbenzylaminen hydriert, die erhaltenen Amine mit einer Verbindung der Formel II

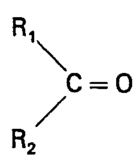

(II)

zu einer Verbindung der Formel III

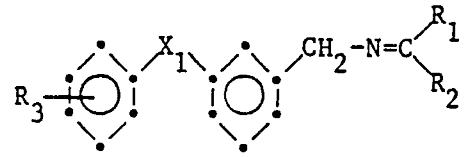

(III)

umsetzt und die Verbindung der Formel III in Gegenwart eines Katalysators der Formel IV

$$(X)^{n+}\ (OY)_n^-$$ (IV)

zu einer Verbindung der Formel I isomerisiert. In den obigen Formeln II und III haben $R_1$, $R_2$, $R_3$ und $X_1$ die unter Formel I angegebene Bedeutung, X stellt ein Alkalimetall- oder Erdalkalimetallion dar, Y bedeutet Alkyl mit 1—12 C-Atomen und n stellt die Ladung des Alkalimetall- oder Erdalkalimetallions dar.

Die 3-Phenoxybenzonitrile resp. 3-Benzylbenzonitrile sowie die Verbindungen der Formeln II und IV sind bekannt oder können nach an sich bekannten Methoden hergestellt werden.

Die katalytische Hydrierung der Nitrile zu den Aminen kann auf an sich bekannte Weise vorgenommen werden, zweckmässig in einem inerten organischen Lösungsmittel und gegebenenfalls in Gegenwart von flüssigem Ammoniak. Beispiele von geeigneten inerten organischen Lösungsmitteln sind: Alkohole mit bis zu 6 C-Atomen, wie Methanol, Aethanol, Propanol, Isopropanol, Butanole und Pentanole; aliphatische und cycloaliphatische Kohlenwasserstoffe, wie n-Pentan, n-Hexan und Cyclohexan; Aethylenglykol- und Diäthylenglykolmono- und dialkyläther mit je 1—4 C-Atomen in den Alkyteilen, wie Aethylenglykol- und Diäthylenglykolmonomethyl- und monoäthyläther, Aethylenglykol- und Diäthylenglykoldimethyl- und diäthyläther. Bevorzugtes Lösungsmittel ist Methanol.

Als Katalysatoren können an sich bekannte Hydrierungskatalysatoren verwendet werden, wie Platin-, Rhodium- und Palladiumkatalysatoren. Bevorzugt verwendet man Nickel-Katalysatoren, besonders Raney-Nickel.

Die Hydrierung wird zweckmässig in geschlossenem System bei einem Druck von etwa 10 bis 200 bar und insbesondere 20 bis 130 bar durchgeführt. Die Hydrierungstemperaturen liegen im allgemeinen zwischen etwa 0 und 150°C und insbesondere zwischen etwa 25 und 80°C.

Nach Beendigung der Umsetzung und dem Entfernen des Katalysators und des Lösungsmittels können die Amine auf übliche Weise isoliert und gereinigt werden, beispielsweise mittels Destillation oder Extraktion mit geeigneten inerten organischen Lösungsmitteln.

Die Umsetzung der Amine mit einer Verbindung der Formel II sowie die Isomerisierung der Verbindungen der Formel III zu Verbindungen der Formel I werden zweckmässig in Gegenwart eines inerten organischen Lösungsmittels vorgenommen. Als inerten organische Lösungsmittel verwendet man insbesondere aprotische organische Lösungsmittel, vor allem aliphatische oder aromatische Kohlenwasserstoffe, aliphatische oder cyclische Aether, Aethylenglykol- und Diäthylenglykol-dialkyläther mit je 1—4 C-Atomen in den Alkylteilen, Alkylnitrile oder Dialkylsulfoxide mit je bis 4 C-Atomen in den Alkylteilen, N,N-Dialkylamide von aliphatischen Monocarbonsäuren mit 1—3 C-Atomen im Säureteil, Alkohole mit mindestens 4 C-Atomen, Sulfolan, Amide der Phosphorsäure und tertiäre Amine. Beispiel solcher Lösungsmittel sind n-Pentan, n-Hexan, n-Heptan, Benzol, Toluol, Xylole, Diäthyläther, Di-n-propyläther, Tetrahydrofuran, Tetrahydropyran, Dioxan, Aethylenglykol- und Diäthylenglykoldimethyläther und -diäthyläther, Dimethylsulfoxid, Acetonitril, Propionitril, Butyronitril, N,N-Dimethylformamid, N,N-Dimethylacetamid, n-Butanol, tert-Butanol, n-Hexanol, Hexamethylphosphorsäuretriamid, Triäthylamin und Pyridin. Es können auch Gemische derartiger Lösungsmittel verwendet werden. Bevorzugte Lösungsmittel sind Dimethylsulfoxid, Dioxan, Tetrahydrofuran, Diäthyläther und insbesondere Toluol.

Die Reaktionstemperaturen für die Umsetzung der 3-substituierten Benzylamine mit der Verbindung der Formel II liegen zweckmässig etwa zwischen 0 und 80°C und besonders zwischen etwa 10 und 60°C. Die Isomerisierung der Verbindungen der Formel III wird bevorzugt bei Temperaturen zwischen etwa 20 und 150°C vorgenommen.

Die Amine und die Verbindung der Formel II werden in mindestens stöchiometrischer Menge eingesetzt. Mit Vorteil verwendet man einen leichten Ueberschuss an Verbindung der Formel II, z.B. einen etwa 5—20%igen Ueberschuss.

Alkylgruppen Y können geradkettig oder verzweigt sein und weisen bevorzugt 1—6 und insbesondere 1—4 C-Atome auf. Besonders bevorzugt stellt Y tert-Butyl dar. X stellt z.B. Lithium, Kalium, Natrium, Magnesium, Calcium oder Barium dar. Bevorzugt bedeutet X ein Alkalimetall, insbesondere Natrium oder Kalium. Besonders bevorzugte Verbindungen der Formel IV sind das Natrium- oder Kaliummethylat, -äthylat, -isopropylat und vor allem das Natrium- oder Kalium-tert-butylat. Die Katalysatoren der Formel IV verwendet man zweckmässig in einer Menge von mindestens 0,1 Mol.%, bezogen auf die Verbindung der Formel III. Bevorzugt sind Mengen von etwa 0,5 bis 15 Mol.%, bezogen auf die Verbindung der Formel III.

Die Verbindungen der Formel I lassen sich auf einfache Weise in hochreine 3-Phenoxybenzaldehyde resp. 3-Benzylbenzaldehyde überführen. Gegenstand der Erfindung ist somit auch die Verwendung erfindungsgemässer Verbindungen der Formel I zur Herstellung von 3-Phenoxybenzaldehyde resp. 3-Benzylbenzaldehyden, indem man die Verbindungen der Formel I mit einer Säure behandelt. Die Ueberführung der Verbindungen der Formel I in die Aldehyde erfolgt bevorzugt in wässrig-organischem Medium. Als Säure verwendet man zweckmässig eine anorganische Säure, wie Salzsäure oder Schwefelsäure. Die Säure wird im allgemeinen in mindestens stöchiometrischer Menge, bezogen auf die Verbindung der Formel I, und bevorzugt im Ueberschuss eingesetzt. Als organische Lösungsmittel setzt man bevorzugt aprotische organische Lösungsmittel der vorerwähnten Art, besonders Toluol, ein. Die bei der Ueberführung in die Aldehyde als Nebenprodukte entstehenden Amine

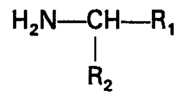

können leicht aus dem Reaktionsmedium abgetrennt und weiter verwertet werden.

Die Zwischenprodukte der Formel III und die Verbindungen der Formel I können gewünschtenfalls auf an sich bekannte Weise isoliert werden, beispielsweise mittels Destillation. Eine solche Zwischenisolierung ist jedoch zur Herstellung der Aldehyde nicht erforderlich. Ein besonderer Vorteil der vorliegenden Erfindung liegt darin, dass sich die Aldehyde auf dem Weg über die neuen Verbindungen der Formel I ohne nennenswerte Verringerung der Ausbeute auch ohne Zwischenisolierung oder Reinigung der Verbindungen der Formeln III und I in hoher Reinheit und unter Vermeidung von aufwendigen Oxidations- oder Reduktionsreaktionen herstellen lässt. Der 3-Phenoxybenzaldehyd kann z.B. auf die eingangs beschriebene Weise in den 3-Phenoxy-$\alpha$-cyanobenzylalkohol übergeführt oder direkt zur Herstellung von bekannten pyrethroidartigen Schädlingsbekämpfungsmitteln verwendet werden.

Beispiel 1

*a) Herstellung von 3-Phenoxybenzylamin*

200 g (1,025 Mol) 3-Phenoxybenzonitril werden zusammen mit 1 Liter Methanol, 200 g flüssigem Ammoniak und 50 g Raney-Nickel in einen Stahlautoklaven eingefüllt. Es wird dann auf 60°C erwärmt und während einer Stunde bei einem Anfangswasserstoffdruck von 120 bar gerührt. Nach dem Ausladen des Autoklaven wird vom Katalysator abfiltriert, das Lösungsmittel wird bei Normaldruck abdestilliert, und der Rückstand wird im Oelpumpenvakuum destilliert. Man erhält 200,3 g (1,01 Mol) 3-Phenoxybenzylamin (98,7%ig), entsprechend einer Ausbeute von 94,7% d.Th.; Sdp. 94°C/3 Pa; $n_D^{20}$ = 1,5946.

Analyse für $C_{13}H_{13}NO$ (Molgewicht 199):

| | | | | |
|---|---|---|---|---|
| berechnet | C 78,36% | H 6,58% | N 7,03% | O 8,03% |
| gefunden | C 78,31% | H 6,61% | N 7,06% | O 8,25%. |

MS-Spektrum: Molekül-Peak 199, Bruchstückmassen 181, 153, 141, 93, 77, 51.
NMR-Spektrum $\tau$[ppm]: 2,6—3,2(m), 6,19(s), 8,58(s) im Verhältnis 9:2:2.

*b) Herstellung von N-Isobutyliden-(3-phenoxybenzylamin)*

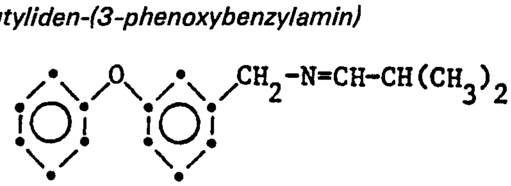

Zu einer Lösung von 99,5 g (0,5 Mol) 3-Phenoxybenzylamin in 100 ml Toluol tropft man unter Rühren innerhalb von 30 Minuten 38 g (0,528 Mol) Isobutyraldehyd zu. Nach Beendetem Zutropfen rührt man noch 20 Minuten bei Raumtemperatur und trennt das gebildete Wasser (ca. 8,5 g) in einem Scheidetrichter ab. Das Toluol mit dem überschüssigen Isobutyraldehyd und Restwasser wird dann im Wasserstrahlvakuum abrotiert, und der Rückstand wird im Oelpumpenvakuum destilliert. Man erhält 124 g (0,49 Mol) N-Isobutyliden-(3-phenoxybenzylamin) als eine farblose, leicht bewegliche Flüssigkeit, entsprechend einer Ausbeute von 97.8% d.Th.; Sdp. 85°C/4 Pa; $n_D^{20}$ = 1,5666.

Analyse für $C_{17}H_{19}NO$ (Molgewicht 253,35):

| | | | | |
|---|---|---|---|---|
| berechnet | C 80,60% | H 7,56% | N 5,53% | O 6,31% |
| gefunden | C 79,91% | H 7,62% | N 5,41% | O 6,52% |

MS-Spektrum: Molekül-Peak 253, Bruchstückmassen 184, 183, 168, 153, 77.
NMR-Spektrum $\tau$ [ppm]: 2,47(d), 2,6—3,2(m), 5,50(s), 7,51(m), 8,87(d) im Verhältnis 1:9:2:1:6.

*c) Herstellung von 3-Phenoxybenzyliden-isobutylamin*

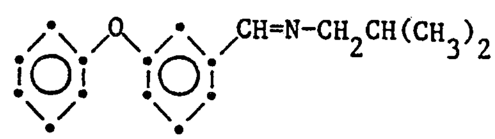

51 g (0,202 Mol) N-Isobutyliden-(3-phenoxybenzylamin) werden in 45 ml Dimethylsulfoxid gelöst und unter Rühren bei Raumtemperatur (20—25°C) auf einmal mit 2 g (0,0179 Mol) Kalium-tert-butylat versetzt. Die Temperatur der Reaktionslösung steigt dabei auf 25—30°C an. Es wird noch 1 Stunde bei Raumtemperatur nachgerührt und dann mit je drei 50 ml Portionen Wasser ausgeschüttelt. Der Rückstand wird in 30 ml Diäthyläther aufgenommen, mit Magnesiumsulfat getrocknet und destilliert. Man erhält 48,5 g (0,192 Mol) 3-Phenoxybenzyliden-isobutylamin, entsprechend einer Ausbeute von 95% d.Th.; Sdp. 83°C/4 Pa; $n_D^{20}$ = 1,5677.

Analyse für $C_{17}H_{19}NO$ (Molgewicht 253,35):

| | | | | |
|---|---|---|---|---|
| berechnet | C 80,60% | H 7,56% | N 5,53% | O 6,31% |
| gefunden | C 80,65% | H 7,73% | N 5,50% | O 6,33%. |

MS-Spektrum: Molekül-Peak 253, Bruchstückmassen 238, 210, 183, 117, 77.
NMR-Spektrum $\tau$ [ppm]: 1,83(s), 2,5—3,1(m), 6,59(dd), 8,00(sept), 9,02(d) im Verhältnis 1:9:2:1:6.

*d) Ueberführung in den 3-Phenoxybenzaldehyd*

Eine Lösung von 40 g (0,158 Mol) 3-Phenoxybenzyliden-isobutylamin in 40 ml Toluol wird unter Rühren mit 20 g 37%iger Salzsäure (ca. 0,2 Mol) und 20 ml Wasser versetzt und während 15 Minuten unter Rückfluss erhitzt. Die organische Phase wird abgetrent, mit 10%iger Natriumhydrogencarbonat-Lösung neutral gewaschen, und das Toluol wird im leichten Wasserstrahlvakuum abrotiert. Die Destillation des Rückstandes ergibt 29,7 g (0,15 Mol) 3-Phenoxybenzaldehyd, entsprechend einer Ausbeute von 94,9% d.Th.; Sdp. 94°C/6 Pa; $n_D^{20}$ = 1,5976.

Der erhaltene 3-Phenoxybenzaldehyd kann direkt, d.h. ohne zusätzliche Reinigungsoperationen, für die Herstellung von pyrethroidartigen Schädlingsbekämpfungsmitteln bzw. des 3-Phenoxy-$\alpha$-cyanobenzylalkohols eingesetzt werden, z.B. nach dem in der deutschen Patentschrift 2.231.312 beschriebenen Verfahren. In der genannten Patentschrift sind auch Eigenschaften und Anwendung der damit erhältlichen insektiziden Wirkstoffe beschrieben.

Auf analoge Weise wird aus

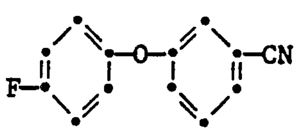

über

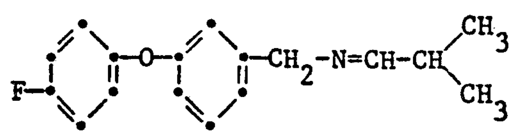

und

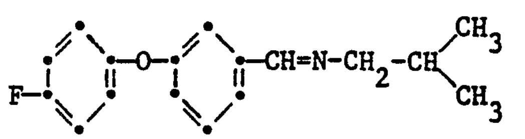

der Aldehyd der Formel

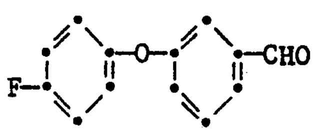

,

aus

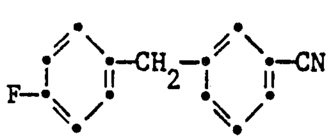

über

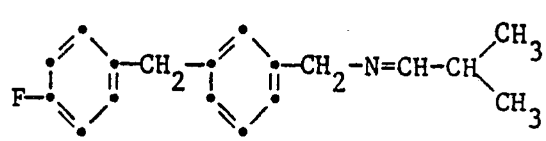

und

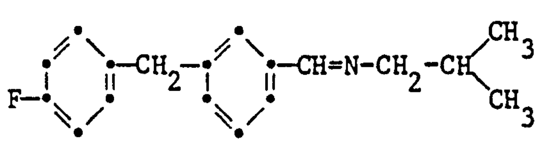

der Aldehyd der Formel

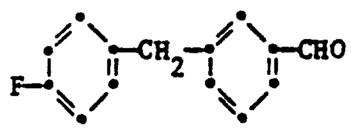

oder

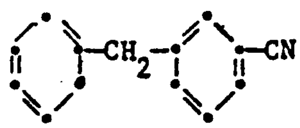

über

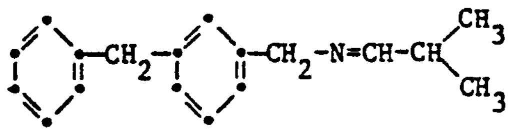

und

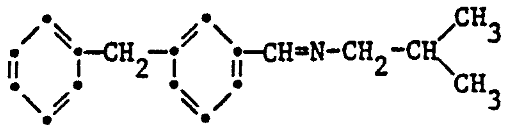

der Aldehyd der Formel

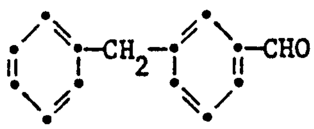

hergestellt.

Beispiel 2

80 g (0,316 Mol) N-Isobutyliden-3-phenoxybenzylamin werden in 80 ml Toluol gelöst, mit 4 g (0,036 Mol) Kalium-tert-butylat versetzt und während 4 Stunden unter Rückfluss gekocht. Nach dem Abkühlen des Reaktionsgemisches gibt man 35 g 37%ige Salzsäure (ca. 0,35 Mol) und 50 ml Wasser hinzu und trennt die toluolische Phase ab. Nach dem Abdestillieren des Toluols erhält man 57 g (0,288 Mol) 3-Phenoxybenzaldehyd, entsprechend einer Ausbeute von 91% d.Th.

Beispiel 3

Es wird wie in Beispiel 1(b) beschrieben vorgegangen, jedoch unter Verwendung von 50 g (0,25 Mol) 3-Phenoxybenzylamin und 30 g (0,348 Mol) Diäthylketon. Nach der Destillation erhält man 62,5 g (0,234 Mol) N-3-Pentyliden-(3-phenoxybenzylamin), entsprechend einer Ausbeute von 93,6% d.Th.; Sdp. 128°C/5 Pa.

Analyse für $C_{18}H_{21}NO$ (Molgewicht 267,37):

| | | | | |
|---|---|---|---|---|
| berechnet | C 80,86% | H 7,92% | N 5,24% | O 5,98% |
| gefunden | C 80,97% | H 7,88% | N 5,36% | O 5,92% |

MS-Spektrum: Molekül-Peak 267, Bruchstückmassen 238, 183, 85, 83.
NMR-Spektrum $\tau$ [ppm]: 2,6—3,3(m), 5,51(s), 7,70(m), 8,90(m) im Verhältnis 9:2:4:6.

60,5 g (0,226 Mol) N-3-Pentyliden-(3-phenoxybenzylamin) werden in 50 ml Toluol gelöst, mit 2 g (0,0179 Mol) Kalium-tert-butylat versetzt und während 4 Stunden unter Rückfluss gekocht. Nach dem Abkühlen des Reaktionsgemisches schüttelt man mit 50 ml Wasser aus und trennt die organische Phase im Scheidetrichter ab. Nach dem Abdestillieren des Toluols im leichten Wasserstrahlvakuum erhält man 56 g (0,21 Mol) 3-Phenoxybenzyliden-3-pentylamin, entsprechend einer Ausbeute von 92,5% d.Th.; Sdp. 125°C/5 Pa.

Analyse für $C_{18}H_{21}No$ (Molgewicht 267,37):

| | | | | |
|---|---|---|---|---|
| berechnet | C 80,86% | H 7,92% | N 5,24% | O 5,98% |
| gefunden | C 81,19% | H 8,08% | N 5,43% | O 6,27%. |

NMR-Spektrum $\tau$ [ppm]: 1,86(s), 2,4—3,1(m), 7,13(quin), 8,38(quin), 9,13(t) im Verhältnis 1:9:1:4:6.

Beispiel 4

Es wird wie in Beispiel 1(b) beschrieben vorgegangen, jedoch unter Verwendung von 38 g (0,528 Mol) n-Butyraldehyd anstelle von Isobutyraldehyd. Nach der Destillation erhält man 65 g (0,257 Mol) N-Butyliden-(3-phenoxybenzylamin), entsprechend einer Ausbeute von 51,4% d.Th.; Sdp. 90—92°C/5 Pa.

Analyse für $C_{17}H_{19}NO$ (Molgewicht 253,35):

| | | | | |
|---|---|---|---|---|
| berechnet | C 80,60% | H 7,56% | N 5,53% | O 6,31% |
| gefunden | C 80,75% | H 7,68% | N 5,28% | O 6,53% |

NMR-Spektrum $\tau$ [ppm]: 2,3(t), 2,6—3,2(m), 5,5(s), 7,7(m), 8,5(m), 9,05(t) im Verhältnis 1:9:2:2:2:3.

Die Isomerisierung des N-Butyliden-(3-phenoxybenzylamins) [60 g, 0,237 Mol] wird auf analoge Weise wie in Beispiel 3 beschrieben vorgenommen. Nach der Destillation erhält man 56,5 g (0,223 Mol) 3-Phenoxybenzyliden-n-butylamin, entsprechend einer Ausbeute von 94,1% d.Th.; Sdp. 134—135°C/13 Pa.

Analyse für $C_{17}H_{19}NO$ (Molgewicht 253,35):

| | | | | |
|---|---|---|---|---|
| berechnet | C 80,60% | H 7,56% | N 5,53% | O 6,31% |
| gefunden | C 80,67% | H 7,72% | N 5,80% | O 6,42%. |

NMR-Spektrum $\tau$ [ppm]: 1,81(s), 2,4—3,1(m), 6,42(t), 8,1—8,8(m), 9,03(t) im Verhältnis 1:9:2:4:3.

Beispiel 5

Es wird wie in Beispiel 1(b) beschrieben vorgegangen, jedoch unter Verwendung von 50 g (0,25 Mol) 3-Phenoxybenzylamin und 25 g (0,255 Mol) Cyclohexanon. Nach der Destillation erhält man 41 g (0,147 Mol) N-Cyclohexyliden-(3-phenoxybenzylamin), entsprechend einer Ausbeute von 58,7% d.Th.; Sdp. 135—136°C/4 Pa.

Analyse für $C_{19}H_{21}NO$ (Molgewicht 279,38):

| | | | | |
|---|---|---|---|---|
| berechnet | C 81,69% | H 7,58% | N 5,01% | O 5,73% |
| gefunden | C 80,81% | H 7,56% | N 5,02% | O 6,12%. |

NMR-Spektrum $\tau$ [ppm]: 2,6—3,2(m), 5,51(s), 7,65(m), 8,33(m) im Verhältnis 9:2:4:6.

50 g (0,179 Mol) N-Cyclohexyliden-(3-phenoxybenzylamin) werden auf die in den vorangehenden Beispielen beschriebene Weise isomerisiert. Nach der Destillation erhält man 46 g (0,165 Mol) 3-Phenoxybenzyliden-cyclohexylamin, entsprechend einer Ausbeute von 92% d.Th.; Sdp. 150°C/10 Pa; Fp. 72—74°C.

Analyse für $C_{19}H_{21}NO$ (Molgewicht 279,38):

| | | | | |
|---|---|---|---|---|
| berechnet | C 81,69% | H 7,58% | N 5,01% | O 5,73% |
| gefunden | C 81,96% | H 7,78% | N 5,13% | O 5,83%. |

NMR-Spektrum $\tau$ [ppm]: 1,78(s), 2,5—3,1(m), 6,84(m), 8,1—8,8(m) im Verhältnis 1:9:1:10.

**Patentansprüche**

1. Eine Verbindung der Formel

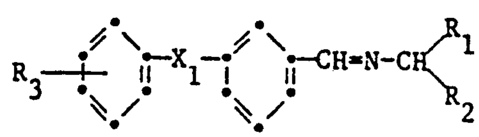

(I)

worin $R_1$ Wasserstoff oder Alkyl und
$R_2$ Alkyl bedeuten, wobei durch
$R_1$ und $R_2$ dargestellte Alkylgruppen zusammen nicht mehr als 12 C-Atome aufweisen oder
$R_1$ und $R_2$ zusammen Alkylen mit 4—11 C-Atomen darstellen,
$R_3$ Wasserstoff, Halogen, Methyl oder Methoxy und
$X_1$ Sauerstoff oder $—CH_2—$ bedeuten.

2. Eine Verbindung gemäss Anspruch 1, worin $R_1$ Wasserstoff oder Alkyl und $R_2$ Alkyl bedeuten und $R_1$ und $R_2$ zusammen nicht mehr als 6 C-Atome aufweisen, oder worin $R_1$ und $R_2$ zusammen Tetramethylen oder Pentamethylen, $R_3$ Wasserstoff, Fluor oder Chlor und $X_1$ Sauerstoff oder $—CH_2—$ bedeuten.

3. Eine Verbindung gemäss Anspruch 1, worin $R_1$ Wasserstoff und $R_2$ $\alpha$-verzweigtes Alkyl mit bis zu 6 C-Atomen, vor allem Isopropyl und $R_3$ Wasserstoff oder p-Fluor und $X_1$ Sauerstoff oder $—CH_2—$ bedeuten.

4. Ein Verfahren zur Herstellung einer Verbindung der Formel I nach Anspruch 1, dadurch gekennzeichnet, dass man eine Verbindung der Formel

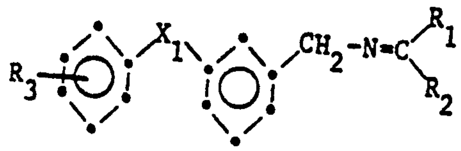

(III)

worin $R_1$ und $R_2$ die unter Formel I angegebene Bedeutung haben, in Gegenwart eines Katalysators der Formel

$$(X)^{n+} \; (OY)^{-}_{n} \qquad (IV)$$

worin X ein Alkalimetall- oder Erdalkalimetallion, Y Alkyl mit 1—12 C-Atomen und n die Ladung des Alkalimetall- oder Erdalkalimetallions bedeuten, isomerisiert.

5. Ein Verfahren nach Anspruch 4, dadurch gekennzeichnet, dass man eine Verbindung der Formel III verwendet, die durch katalytische Hydrierung von 3-Phenoxybenzonitrilen resp. 3-Benzylbenzonitrilen und anschliessende Umsetzung der erhaltenen Amine mit einer Verbindung der Formel II

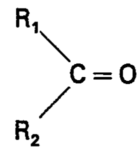

(II)

worin $R_1$ und $R_2$ die unter Formel I angegebene Bedeutung haben, erhalten wird.

6. Ein Verfahren nach Anspruch 4, dadurch gekennzeichnet, dass man die Isomerisierung in einem inerten organischen Lösungsmittel bei einer Temperatur zwischen etwa 20 und 150°C vornimmt.

7. Ein Verfahren nach Anspruch 4 oder 6, dadurch gekennzeichnet, dass man eine Verbindung der Formel IV verwendet, worin X ein Alkalimetallion, besonders das Natrium- oder Kaliumion, und Y Alkyl mit 1—4 C-Atomen, besonders tert-Butyl, darstellen.

8. Verwendung einer Verbindung der Formel I nach Anspruch 1 zur Herstellung von 3-Phenoxybenzaldehyden resp. 3-Benzylbenzaldehyden, dadurch gekennzeichnet, dass man eine Verbindung der Formel I mit Säure behandelt.

## Claims

1. A compound of the formula

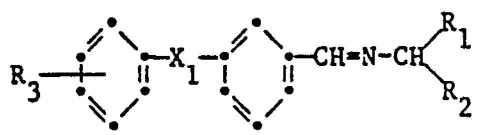

(I)

in which

$R_1$ is hydrogen or alkyl,

$R_2$ is alkyl, with alkyl groups denoted by $R_1$ and $R_2$ together not containing more than 12 C atoms, or $R_1$ and $R_2$ together being alkylene having 4—11 C atoms,

$R_3$ is hydrogen, halogen, methyl or methoxy, and

$X_1$ is oxygen or $—CH_2—$.

2. A compound according to Claim 1, wherein $R_1$ is hydrogen or alkyl, and $R_2$ is alkyl, with $R_1$ and $R_2$ together not containing more than 6 C atoms, or $R_1$ and $R_2$ together being tetramethylene or pentamethylene, $R_3$ is hydrogen, fluorine or chlorine, and $X_1$ is oxygen or $—CH_2—$.

3. A compound according to Claim 1, wherein $R_1$ is hydrogen, and $R_2$ is $\alpha$-branched alkyl having up to 6 C atoms, particularly isopropyl, $R_3$ is hydrogen or p-fluorine, and $X_1$ is oxygen or $—CH_2—$.

4. A process for producing a compound of the formula I according to Claim 1, characterised in that a compound of the formula

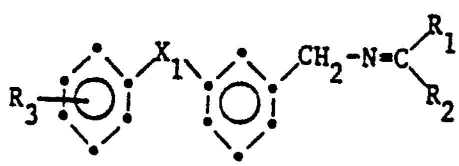

in which $R_1$ and $R_2$ have the meanings given under the formula I, is isomerised in the presence of a catalyst of the formula

$(X)^{n+}\ (OY)^-_n$ (IV)

wherein X is an alkali metal ion or alkaline-earth metal ion, Y is alkyl having 1—12 C atoms, and n is the charge of the alkali metal ion or alkaline-earth metal ion.

5. A process according to Claim 4, characterised in that there is used a compound of the formula III which is obtained by catalytic hydrogenation of 3-phenoxybenzonitriles or 3-benzylbenzonitriles, and subsequent reaction of the resulting amines with a compound of the formula II

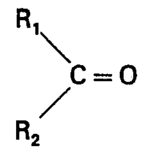

(II)

in which $R_1$ and $R_2$ have the meanings given under the formula I.

6. A process according to Claim 4, characterised in that isomerisation is performed in an inert organic solvent at a temperature between about 20 and 150°C.

7. A process according to Claim 4 or 6, characterised in that there is used a compound of the formula IV in which X is an alkali metal ion, particularly the sodium or potassium ion, and Y is alkyl having 1—4 C atoms, particularly tert-butyl.

8. Use of a compound of the formula I according to Claim 1 for producing 3-phenoxybenzaldehydes or 3-benzylbenzaldehydes, characterised in that a compound of the formula I is treated with acid.

**Revendications**

1. Composé de formule

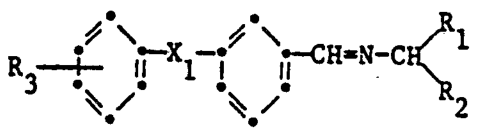

(I)

où $R_1$ représente un hydrogène ou un alcoyle et

$R_2$ un alcoyle, les groupes alcoyle représentés par $R_1$ et $R_2$ ne présentant ensemble pas plus de 12 atomes de carbone, ou

$R_1$ et $R_2$ représentent ensemble un alcoylène ayant de 4 à 11 atomes de carbone,

$R_3$ un hydrogène, un halogène, un méthyle ou un méthoxy et

$X_1$ un oxygène ou $—CH_2—$.

2. Composé selon la revendication 1 où $R_1$ représente un hydrogène ou un alcoyle et $R_2$ un alcoyle et où $R_1$ et $R_2$ ne présentent ensemble, pas plus de 6 atomes de carbone, ou bien où $R_1$ et $R_2$ représentent ensemble un tétraméthylène ou un pentaméthylène et $R_3$ un hydrogène, un fluor ou un chlore et $X_1$ un hydrogène ou $—CH_2—$.

3. Composé selon la revendication 1, où $R_1$ représente un hydrogène et $R_2$ un alcoyle ramifié en $\alpha$ avec jusqu'à 6 atomes de carbone, surtout l'isopropyle, et $R_3$ représente un hydrogène ou un p-fluor et $X_1$ un oxygène ou $—CH_2—$.

4. Procédé de préparation d'un composé de formule I selon la revendication 1, caractérisé en ce qu'on isomérise un composé de formule

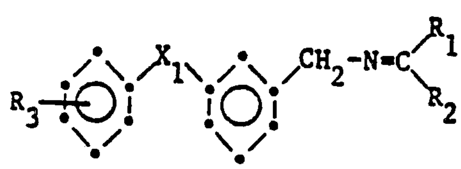

où $R_1$ et $R_2$ ont la signification donnée pour la formule I, en présence d'un catalyseur de formule

$(X)^{n+}\ (OY)^-_n$ (IV)

où X représente un ion de métal alcalin ou alcalinoterreux, Y un alcoyle ayant de 1 à 12 atomes de carbone et n la charge de l'ion de métal alcalin ou alcalinoterreux.

5. Procédé selon la revendication 1, caractérisé en ce qu'on composé de formule III que l'on obtient par hydrogénation catalytique de 3-phénoxybenzonitrile ou de 3-benzylbenzonitrile puis par réaction de l'amine obtenue avec un composé de formule II

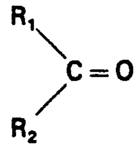

(II)

où $R_1$ et $R_2$ ont la signification donnée pour la formule I.

6. Procédé selon la revendication 4, caractérisé en ce qu'on effectue l'isomérisation dans un solvant organique inerte à une température comprise entre environ 20 et 150°C.

7. Procédé selon l'une des revendications 4 et 6, caractérisé en ce qu'on utilise un composé de formule IV où X représente un ion de métal alcalin, en particulier l'io sodium ou potassium, et Y un alcoyle ayant de 1 à 4 atomes de carbone, en particulier le tert-butyle.

8. Application d'un composé de formule I selon la revendication 1 à la préparation de 3-phénoxybenzaldéhydes ou de 3-benzylbenzaldéhydes, caractérisé en ce qu'on traite un composé de formule I avec un acide.